(19) Europäisches Patentamt
European Patent Office
Office européen des brevets

(11) **EP 3 838 128 A1**

(12) **EUROPEAN PATENT APPLICATION**

(43) Date of publication:
**23.06.2021 Bulletin 2021/25**

(21) Application number: **19216506.6**

(22) Date of filing: **16.12.2019**

(51) Int Cl.:
*A61B 5/024* (2006.01)    *A61B 5/11* (2006.01)
*A61B 5/1455* (2006.01)    *A61B 5/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(71) Applicant: **Koninklijke Philips N.V.**
**5656 AG Eindhoven (NL)**

(72) Inventors:
• **WANG, Wenjing**
  **5656 AE Eindhoven (NL)**
• **KIRENKO, Ihor Olehovych**
  **5656 AE Eindhoven (NL)**

(74) Representative: **Philips Intellectual Property & Standards**
**High Tech Campus 5**
**5656 AE Eindhoven (NL)**

(54) **DEVICE AND METHOD FOR DETERMINING A VITAL SIGN OF A SUBJECT**

(57)    The present invention relates to a device and method for determining a vital sign of a subject. The device comprises a first camera (11) configured to acquire first images of the subject in a visible portion of the spectrum, a second camera (12) configured to acquire second images of the subject in an infrared portion of the spectrum, a visible light source (13) configured to emit visible light, and an infrared light source (14) configured to emit infrared light, which are all arranged on the same surface side of the device. A processor (16) can determine SpO2 of the subject from the red portion of the spectrum of the first images and the infrared portion of the second images, wherein the light emitted by the visible light source is controlled or filtered so that light in the red portion of the spectrum is emitted and/or the light sensed by the first camera is filtered so that light in the red portion of the spectrum is sensed and represented by the first images.

FIG.1

**Description**

FIELD OF THE INVENTION

The present invention relates to a device and method for determining a vital sign of a subject.

BACKGROUND OF THE INVENTION

Vital signs of a person, for example the heart rate (HR), the respiration rate (RR) or the arterial blood oxygen saturation, serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular pulse wave traveling through the body of a subject with every heartbeat.

Photoplethysmography (PPG) is an optical measurement technique that evaluates a time-variant change of light reflectance or transmission of an area or volume of interest. PPG is based on the principle that blood absorbs light more than surrounding tissue, so variations in blood volume with every heart beat affect transmission or reflectance correspondingly. Besides information about the heart rate, a PPG waveform can comprise information attributable to further physiological phenomena such as the respiration. By evaluating the transmittance and/or reflectivity at different wavelengths (typically red and infrared), the blood oxygen saturation can be determined.

Conventional pulse oximeters (also called contact PPG device herein) for measuring the heart rate and the (arterial) blood oxygen saturation (also called SpO2) of a subject are attached to the skin of the subject, for instance to a fingertip, earlobe or forehead. Therefore, they are referred to as 'contact' PPG devices. Although contact PPG is regarded as a basically noninvasive technique, contact PPG measurement is often experienced as being unpleasant and obtrusive, since the pulse oximeter is directly attached to the subject and any cables limit the freedom to move and might hinder a workflow.

Recently, non-contact, remote PPG (rPPG) devices (also called camera rPPG device herein) for unobtrusive measurements have been introduced. Remote PPG utilizes light sources or, in general radiation sources, disposed remotely from the subject of interest. Similarly, also a detector, e.g., a camera or a photo detector, can be disposed remotely from the subject of interest. Therefore, remote photoplethysmographic systems and devices are considered unobtrusive and well suited for medical as well as non-medical everyday applications. The principles of rPPG as well as methods and devices using rPPG are e.g. disclosed in W. Wang, A. C. den Brinker, S. Stuijk, and G. de Haan, "Algorithmic principles of remote-PPG," IEEE Transactions on Biomedical Engineering, vol. 64, no. 7, pp. 1479 - 1491, 2017.

Remote PPG systems have been implemented by the applicant as application program ("APP") on hand-held devices (e.g. mobile phone, pad or tablet) to contactless monitor human vital signs and improve human life. It provides daily-based measurement of pulse-rate (variability), respiratory rate for users to track their body conditions and manage their life-styles. It is portable, light-weighted and easy-to-use, not restricted to time and location. This vital signs camera APP led to a tremendous amount of downloads (i.e. a top download in the healthcare APP category). Since then, the competition in the market of vital signs APP has become rather intensive, i.e. many companies and startups started to work on this topic and made claims and APP releases.

SUMMARY OF THE INVENTION

It is an object of the present invention to further improve an rPPG device and method, in particular implemented as hand-held device or APP, with respect to reliability and robustness as well as usability.

In a first aspect of the present invention a device for determining a vital sign of a subject is presented comprising:

- a first camera configured to acquire first images of the subject in a visible portion of the spectrum,
- a second camera configured to acquire second images of the subject in an infrared portion of the spectrum,
- a visible light source configured to emit visible light,
- an infrared light source configured to emit infrared light,
- a processor configured to determine a vital sign of the subject at least from the acquired first images,

wherein the processor is configured to determine SpO2 of the subject from the red portion of the spectrum of the first images and the infrared portion of the second images,

wherein, in case SpO2 shall be determined as vital sign, the light emitted by the visible light source is controlled or filtered so that light in the red portion of the spectrum is emitted and/or the light sensed by the first camera is filtered so that light in the red portion of the spectrum is sensed and represented by the first images, and
wherein the first camera, the second camera, the visible light source and the infrared light source are arranged on the same surface side of the device.

In a further aspect of the present invention a method for determining a vital sign of a subject is presented comprising:

- controlling a first camera to acquire first images of the subject in a visible portion of the spectrum,
- controlling a second camera to acquire second images of the subject in an infrared portion of the spectrum,
- controlling a visible light source to emit visible light,
- controlling an infrared light source, arranged on the same surface side of a device as the first camera, the second camera and the visible light source, to emit infrared light, and
- determining SpO2 of the subject from the red portion of the spectrum of the first images and the infrared portion of the second images, wherein the light emitted by the visible light source is controlled or filtered so that light in the red portion of the spectrum is emitted and/or the light sensed by the first camera is filtered so that light in the red portion of the spectrum is sensed and represented by the first images.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, computer program and medium have similar and/or identical preferred embodiments as the claimed device, in particular as defined in the dependent claims and as disclosed herein.

The present invention is based on the idea to measure vital signs from both the visible light and invisible light cameras of a device, in particular two frontal cameras of a hand-held device, such as a smartphone, pad, tablet or watch. The present invention enables the measurement of SpO2 that cannot be measured by the existing vital signs APP that uses an RGB camera only. It also enables the measurement of vital signs in full-darkness (night mode), broadening its application scope (e.g. sleep monitoring). Furthermore, it may further improve the robustness of vital signs extraction in terms of front-end (e.g. face detection) and core algorithms (e.g. PPG extraction) by using 3D information of stereoscopic image from dual cameras and more wavelength channels.

The present invention thus overcomes the limitations of the current vital signs APP, by which the possible vital signs that can be measured in visible light are restricted to pulse-rate (variability) and respiratory rate, but which does not allow measuring the true/absolute SpO2 level, as it requires at least one NIR wavelength to do calibration. Further, the current vital signs APP only works in the visible light condition, which restricts its application scope. Some scenarios that desire vital signs monitoring, but then in full-darkness cannot profit from this APP, such as sleep monitoring and driver monitoring in automotive applications.

A number of benefits are thus achieved: (i) it is sensitive to both RGB (R-wavelength) and NIR, thus enables the SpO2 measurement that current vital signs APP do not have; (ii) it can work in full-darkness by using the NIR-camera, which can measure, at least, pulse-rate (variability) and respiratory rate in the night vision (not possible for the current vital signs APP); (iii) it provides stereoscopic images of two cameras with different views, which can increase the robustness of the front-end (e.g. face detection/tracking, image registration, video stabilization); (iv) it increases the robustness of vital signs extraction (i.e. core algorithms), by using more wavelengths for distortion suppression or using 3D information (e.g. depth) from stereoscopic image for 3D respiratory motion estimation (e.g. respiration-induced distance changes between chest and camera).

Thus, the present invention increases the number of measured vital signs (e.g. SpO2) that can be measured from a hand-held device. Further, it broadens its application scope (in particular to full-darkness scenarios) and improves the robustness of the existing vital signs measurement.

According to an embodiment the device further comprises a controller configured to control the visible light source to emit, in case SpO2 shall be determined as vital sign, light in the red portion of the spectrum. For instance, an OLED screen, representing the visible light source, to emit red light to create a narrow-band sensing (light source modification).

In another embodiment, a red filter (in particular a narrow-band red filter) may be arranged in front of the first camera (optical modification), which is preferably configured to be switched on or off or to be arranged in front of or to be removed from the first camera. Thus, additional hardware is used according to these embodiments.

According to another embodiment the processor is configured to extract the red portion of the spectrum from the first images. Thus, additional software is used according to this embodiment.

The device may further comprise a controller configured to control the infrared light source. The controller may be configured to control the infrared light source to emit a grid pattern onto the subject, wherein the processor is configured to derive 3D information from the second images and to use the derived 3D information for detection and/or tracking of a skin region of the subject and/or for detection of movement of a body portion of the subject's body. In an alternative embodiment the processor is configured to receive 3D information of the subject and to use the derived 3D information for detection and/or tracking of a skin region of the subject and/or for detection of movement of a body portion of the subject's body caused by respiration of the subject and/or ballistocardiographic motion. Thus, with use of the 3D information the accuracy and reliability of a detected vital sign can be increased.

The 3D information may further be used to register (for the reduction of parallax) first and second images before using the first and/or second images to determine a vital sign. This improves the accuracy of the determination of the vital sign. In an embodiment for registration of the first and second images the processor is configured to use a global linear-model based pixel transformation or local non-linear block-to-block or pixel-to-pixel based pixel interpolation.

The controller may be configured to adjust the spectrum, amplitude and/or modulation frequency of the light emitted by the visible light source and/or the infrared light source based on a quality metric of the derived vital sign. Thus, a kind of feedback loop is used to provide an appropriate control of the visible light source and/or the infrared light source to further improve the quality of the derived vital sign.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of a first embodiment of a device according to the present invention,
Fig. 2 shows an implementation of a device according to the present invention in the form of a smartphone,
Fig. 3 shows a schematic diagram of a second embodiment of a device according to the present invention,
Fig. 4 shows a schematic diagram of a third embodiment of a device according to the present invention, and
Fig. 5 shows a flowchart of an embodiment of a method according to the present invention.

DETAILED DESCRIPTION OF THE INVENTION

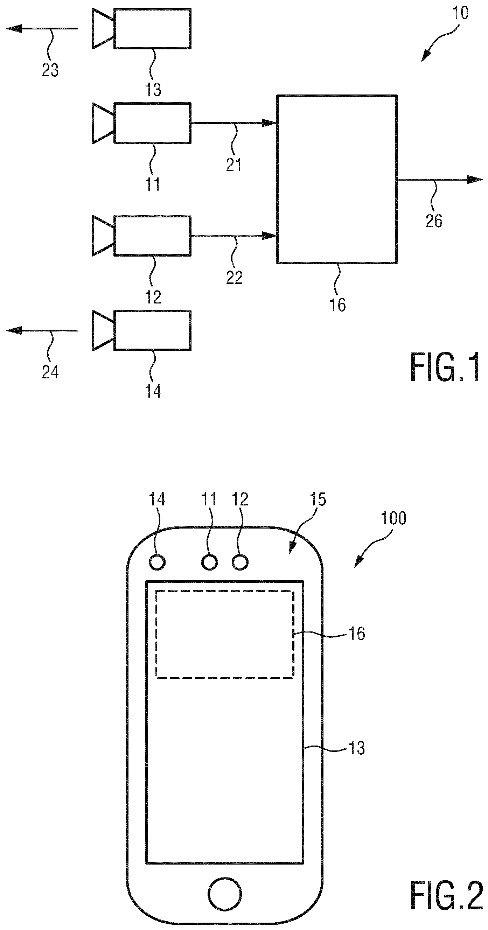

The general concept of the present invention will be explained by reference to Figs. 1 and 2. Fig. 1 shows a schematic diagram of a first embodiment of a device 10 according to the present invention. Fig. 2 shows an implementation of the device 10 according to the present invention in the form of a smartphone 100. The device 10 is configured to determine a vital sign of a subject, such as the pulse rate, pulse rate variability, respiration rate or blood oxygen saturation (SpO2), in a robust and contactless manner. The device 10 is particularly configured for use in everyday situations so that a person can easily and quickly monitor its own body situation. Preferably, a common device like a smartphone, watch or pad shall thus be made usable for this purpose. The proposed method can thus e.g. be implemented in the form of an APP that is running on a conventional handheld/portable device.

The device 10 comprises a first camera 11 for acquiring first images 21 of the subject in a visible portion of the spectrum and a second camera 12 for acquiring second images 22 of the subject in an infrared portion of the spectrum. The first images 21 and the second images are synchronously acquired. The first camera may be a conventional RGB camera as e.g. typically used in a smartphone for taking images, which typically samples at 460, 580 and 660 nm. The second camera 12 may be a near-infrared (NIR) camera, which may be a monochrome camera sampling at 940 nm.

The device 10 further comprises a visible light source 13 for emitting visible light 23. The visible light source 13 may e.g. be an LED or OLED display as typically used in a smartphone for displaying all kinds of information and for operating as user interface in the form of touch screen. In another embodiment, it may be a simple screen only without having the function of a touch screen or a simple light source, e.g. a flashlight, or an array of light sources.

The device 10 further comprises an infrared light source 14 for emitting infrared light 24. The infrared light source 14 may e.g. be an NIR LED or an array of NIR LEDs.

As particularly shown in Fig. 2, the first camera 11, the second camera 12, the visible light source 13 and the infrared light source 14 are arranged on the same surface side 15 of the device 10, i.e. on the surface side 15 that faces the subject when the device is used for determining a vital sign. These elements may thus be integrated into the surface 15 or mounted on top of the surface 15.

The device 10 further comprises a processor 16 for determining a vital sign 26 of the subject at least from the acquired first images. The processor 16 may be a conventional processing logic or circuitry, e.g. as typically used in a smartphone. The processor 16 is preferably programmable and configured via soft- and/or hardware to carry out desired functions. For determining one or more vital signs, conventional technology is used. For instance, known methods for determining vital signs by use of remote PPG, as explained in the introductory portion and described in many publications may be used. Further, period motions of e.g. the chest wall may be detected from the first and/or second images 21, 22 to determine the respiration rate.

The processor 16 is particularly configured to determine SpO2 of the subject from the red portion of the spectrum of the first images 21 and the infrared portion of the second images 22. For determining SpO2 a conventional ratio-of-ratio based approach as e.g. described in WO 2015/197383 A1 can be used, which description of the ratio-of-ratio based approach is herein incorporated by reference. From the measured PPG amplitude (caused by pulsatile blood in arteries) at two distinct wavelengths (red and infrared) the ratio between the PPG amplitudes (DC normalized) of the two wavelengths give the SpO2. Alternatively, an adaptive blood volume pulse (APBV) signature based approach may be used as e.g. described in M. van Gastel, S. Stuijk and G. de Haan, "New principle for measuring arterial blood oxygenation, enabling motion-robust remote monitoring", Nature Scientific Reports, Nov. 2016, which description of APBV is herein incorporated by reference.

In case SpO2 shall be determined as vital sign, the light 23 emitted by the visible light source 13 is controlled or filtered so that light in the red portion of the spectrum is emitted and/or the light sensed by the first camera 11 is filtered so that light in the red portion of the spectrum is sensed and represented by the first images 21. Thus, different options are available to provide that the first images 21 particularly represent light in the red wavelength channel when SpO2 shall be determined as vital sign. These options will be explained in more detail below.

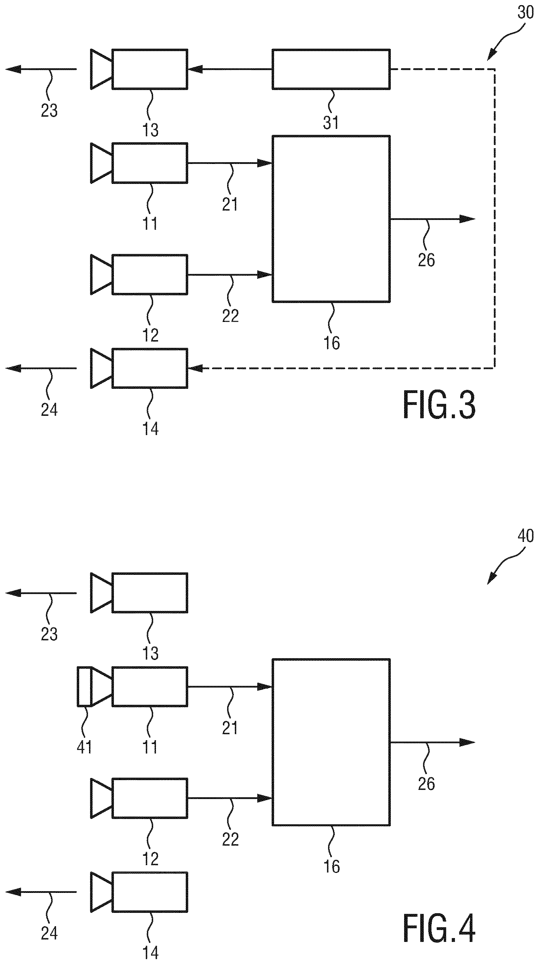

Fig. 3 shows a schematic diagram of a second embodiment of a device 30 according to the present invention. In this embodiment, the device 30 further comprises an optional controller 31 for controlling the visible light source 13 to emit, in case SpO2 shall be determined as vital sign, light in the red portion of the spectrum. The controller 41 may be implemented in soft- and/or hardware. In an embodiment, the processor 16 may be configured to operate as controller as well so that no separate hardware element is provided in addition to the controller.

The controller 31 may, additionally or alternatively, be configured to control the infrared light source 14 to adjust the wavelength of the emitted infrared light.

Further, the controller 31 may operate as lighting adjustment unit to adjust the spectrum of the visible light source 13 and/or the spectrum of infrared light source 14 to provide an illumination spectrum aligned with the wavelengths of the first camera 11 and/or the second camera 12 required for vital signs extraction. For instance, the visible light source 13 and/or the infrared light source 14 can be modulated with certain frequency and/or amplitude in time to improve the robustness of vital signs measurement. The adjustment of the spectrum, amplitude and/or modulation frequency of the light emitted by the visible light source 13 and/or the infrared light source 14 may hereby be based on a quality metric (e.g. signal to noise ratio, robustness, accuracy, etc.) of the derived vital sign, i.e. as a kind of feedback loop.

The controller 31 may further be configured to control the infrared light source 13 to emit a grid pattern onto the subject. In this case, the processor 16 is able to derive 3D information from the second images 22. The 3D information may then be used for detection and/or tracking of a skin region (e.g. the face, the forehead, the cheek, etc.) of the subject (from which PPG signals are then detected and evaluated for determining a vital sign). The 3D information may further be used for detection of movement of a body portion of the subject's body caused by respiration of the subject (from which respiratory information like the respiration rate can then be derived, i.e. to support or enable respiratory motion estimation by estimating respiration-induced distance changes between chest and camera) and/or ballistocardiographic motion.

For detection and/or tracking of a skin region various approaches may be used. These approaches include a machine learning based face detection (e.g. supervised learning like Viola-Jones or convolutional neural network (CNN) based face detector), living-skin detection (remote-PPG based), depth based face detection (the two cameras 11, 12 can provide depth information, with the assumption that face is the closest object to the camera), or the 3D object structure provided by the NIR camera 12, and the combination of these approaches.

In another embodiment, 3D information can be obtained from stereoscopic images of the scene, i.e. from images from two different camera views. Such stereoscopic images may be acquired by two separate cameras or a stereoscopic imaging unit, as provided in such an embodiment of the device, or even by the two cameras 11, 12.

In another embodiment, the processor 16 may be configured to register first and second images using the 3D information before using the first and/or second images to determine a vital sign. A global linear-model based pixel transformation or local non-linear block-to-block or pixel-to-pixel based pixel interpolation may hereby be used for registration of the first and second images. The first and second images 21, 22 from the two cameras 11, 12 are preferably registered in a same image plane to reduce the parallax for skin segmentation and signal extraction. This stabilize the acquired images during handheld measurement and further improves the quality of the finally extracted vital sign(s).

Fig. 4 shows a schematic diagram of a third embodiment of a device 40 according to the present invention. In this embodiment, the device 50 further comprises a red filter 41 arranged in front of the first camera 11. The red filter 41 can preferably be switched on or off (e.g. by the controller 31 shown in Fig. 3 or the processor 16). Alternatively, it may be configured such that it can be arranged in front of or removed from the first camera, e.g. via a mechanism allowing manual or automatic change of the position of the filter 41 (e.g. via a sliding or rotational movement).

In an embodiment, only the red channel (R-channel) of the first camera 11 is used for SpO2 extraction. If the second camera 12 is also multi-spectral, other NIR wavelengths can also be used for SpO2 extraction.

The R-channel of an RGB camera usually includes a large portion of a greed component (G-component). To have a better separation between R and G for SpO2 measurement, a notch filter emphasizing the R-channel may be provided in front of the first camera. Alternatively, the green and blue channels of the first camera 11 can still be used as additional sensors to support the measurement (e.g. to clean or de-noise the signal, remove artifacts, segment skin (using color information), diagnose the lighting condition, etc.), but preferably they are not directly involved in the SpO2 extraction/calibration.

Based on the detected skin (e.g. face), global wavelength signals may be derived from the whole skin area or multiple local wavelength signals can be generated from densely segmented skin areas, exploiting spatial redundancy. Further, in case of such a multi-site measurement, local SpO2 values may be selected or combined (e.g. averaged) to obtain a final vital sign.

If the visible light source 13 is configured as display, as shown in Fig. 2, the (instantaneous) vital sign(s), e.g. SpO2 results, can be displayed as values and/or traces). Further, additional information may be issued, such as a quality metric to indicate the reliability of the measurement and/or an alarm (e.g. video based, audio based, flashlight based, or vibration based) if critical values of a vital sign (e.g. low SpO2 values) are detected.

If the two images 21, 22 from the two cameras 11, 12 are not registered, an accurate skin detector and tracker may be used to detect the skin in the two separate images simultaneously and to ensure that the signals extracted from the two images exactly correspond to the same skin area.

In addition to the core algorithms used for vital signs extraction, other preprocessing (e.g. band-pass filtering, smoothing, de-trending, etc.) or post-processing (e.g. mean filtering, median filtering, etc.) steps may be used to improve the quality of the vital signs extraction.

As mentioned above, there are various options for determining SpO2. One option is to use the ratio-of-ratios method using a ratio of relative pulsatilities, i.e. the normalized amplitude of the pulsatile signal (AC/DC) in different wavelength channels, in particular in the red channel and the infrared channel. Another option is to make use of the APBV method extract a SpO2 value from two or more different combinations of three wavelength channels, e.g. from $[\lambda1, \lambda2]$, from $[\lambda1, \lambda3]$, and/or from $[\lambda1, \lambda2, \lambda3]$. In the following, some basic considerations with respect to the APBV method shall be briefly explained.

Instead of extracting features from the PPG waveforms, APBV determines SpO2 indirectly based on the signal quality of the pulse signals extracted with SpO2 'signatures'. This procedure can mathematically be described as:

$$SpO_2 = \underset{SpO_2 \in \mathbf{SpO_2}}{\operatorname{argmax}} \; SNR\left( \overbrace{k\vec{P}_{bv}(SpO_2)[\mathbf{C_n C_n^T}]^{-1} \mathbf{C_n}}^{\vec{W}_{PBV}} \right),$$

where $\mathbf{C_n}$ contains the DC-normalized color variations and scalar k is chosen such that $\vec{W}_{PBV}$ has unit length. The SpO2 signatures compiled in $\vec{P}_{bv}$, can be derived from physiology and optics. Assuming identical cameras the PPG amplitudes of N cameras can be determined by:

$$\vec{P}_{bv} = \left\| \left[ \begin{array}{c} (\frac{AC}{DC})^1 \\ (\frac{AC}{DC})^2 \\ \vdots \\ (\frac{AC}{DC})^N \end{array} \right] \right\| = \left\| \left[ \begin{array}{c} \frac{\int_\lambda I(\lambda)F^1(\lambda)C(\lambda)PPG(\lambda)d\lambda}{\int_\lambda I(\lambda)F^1(\lambda)C(\lambda)\rho_s(\lambda)d\lambda} \\ \frac{\int_\lambda I(\lambda)F^2(\lambda)C(\lambda)PPG(\lambda)d\lambda}{\int_\lambda I(\lambda)F^2(\lambda)C(\lambda)\rho_s(\lambda)d\lambda} \\ \vdots \\ \frac{\int_\lambda I(\lambda)F^N(\lambda)C(\lambda)PPG(\lambda)d\lambda}{\int_\lambda I(\lambda)F^N(\lambda)C(\lambda)\rho_s(\lambda)d\lambda} \end{array} \right] \right\|.$$

Here the PPG amplitude spectrum, $PPG(\lambda)$ can be approximated by a linear mixture of the light absorption spectra from the two most common variants of the main chromophore in arterial blood, hemoglobin; oxygenated (HbO2) and reduced (Hb):

$$PPG(\lambda) \approx \epsilon_{Hb}(\lambda)c_{Hb} + \epsilon_{HbO_2}(\lambda)c_{HbO_2} = (1 - SaO_2)\epsilon_{Hb}(\lambda) + SaO_2\epsilon_{HbO_2}(\lambda)$$
$$= \epsilon_{Hb}(\lambda) + SaO_2[\epsilon_{HbO_2}(\lambda) - \epsilon_{Hb}(\lambda)],$$

where it is assumed that the optical path length differences are negligible for $600 < \lambda < 1000$ nm and SaO2 $\in [0, 1]$. It is recognized that the wavelength-dependent effect of scattering could render this assumption invalid. When using two wavelengths, the ratio-of-ratios parameter and the ratio of APBV parameter $\vec{P}_{bv}$ coincide. The wavelength selection may be based on three criteria: 1) the desire to measure oxygen saturation in darkness ($\lambda > 700$ nm) for clinical applications, 2) have a reasonable SpO2 contrast, and 3) wavelengths within the spectral sensitivity of the camera. The idea to use three instead of the common two wavelengths used in pulse-oximetry was motivated by the improved robustness of the SpO2 measurement by a factor of two. This can be explained by how motion affects the PPG waveforms when measured with a camera. Since motion-induced intensity variations are equal for all wavelengths, suppression of these artifacts is possible for the APBV method if the pulse signature $\vec{P}_{bv}$, is not equal to this motion signature, which can be described as a vector with equal weights. More details can be found in the above-cited paper M. van Gastel, S. Stuijk and G. de Haan, "New principle for measuring arterial blood oxygenation, enabling motion-robust remote monitoring", Nature Scientific Reports, Nov. 2016.

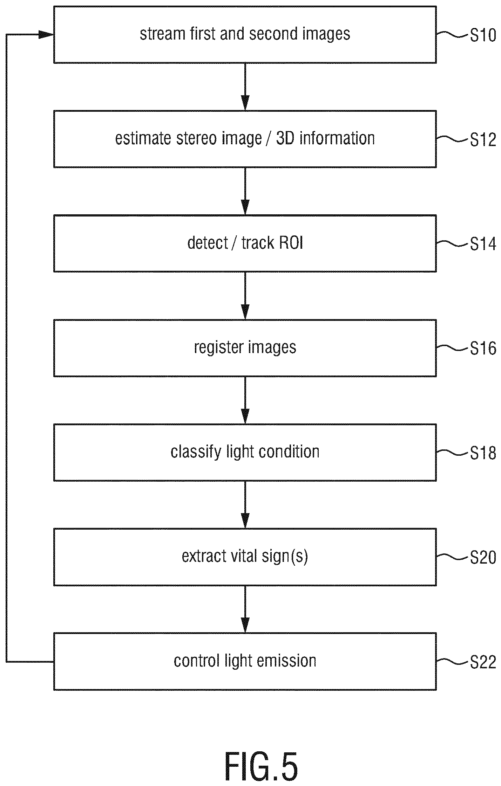

Fig. 5 shows a flowchart of an embodiment of a method 200 according to the present invention. The method 200 includes the following steps:

Step S10: Stream both the RGB image sequence (the first images 21) and NIR image sequence (the second images 22) from both the RGB camera (the first camera 11) and NIR camera (the second camera 12) synchronously such that they sample the scene at the same time.

Step S12: Use an RGB image and the simultaneously acquired NIR image to estimate a stereoscopic image (or disparity map) using their epipolar geometry from different camera views, which contains 3D information (e.g. depth) of the scene. Another way to extract 3D information is using the NIR image using a projected NIR-light grid.

Step S14: Use the 3D information to detect and track the skin region of interest (ROI).

Step S16: Use 3D information to register the skin ROI from two camera views and stabilize the camera motion due to hand movement in the hand-held mode.

Step S18: Classify the light condition into visible (day mode) or invisible (night mode) based on image analysis.

Step S20: In the visible light condition, extract all vital signs (e.g. pulse rate (variability), respiratory rate, SpO2) from the skin ROI. In the invisible light condition, extract the vital signs except SpO2. When measuring the respiratory signal, 3D information from the stereoscopic image can be used to measure the respiration-induced distance changes.

Step S22: Use the measured vital signs, via a quality metric, to control/adjust the NIR LED (infrared light source 14 and/or screen OLED (visible light source 13), e.g. the light spectrum, modulation frequency and amplitude, to improve/optimize the vital signs monitoring in a feedback loop.

In summary, according to embodiments of the present invention the frontal RGB camera and (single-wavelength) IR camera of a smartphone (or other handheld device) may be used for vital signs extraction. Such an IR camera is equipped on latest smartphones for face anti-spoofing purpose (face unlock). Since the RGB camera of the smartphone is a broad-band camera with strong spectrum overlap between RGB filters (e.g. R channel is not purely red, contain lots of green), a narrow-band filter may be added in front of the RGB camera to make it a narrow-band red sensor and use it together with the IR camera for SpO2 measurement.

The frontal IR camera of the smartphone together with the IR illuminator is not a regular IR setup as used in other applications. It may be used as a 3D structural light setup which projects an IR grid pattern on the face and body surface. From the grid mesh 3D information can be derived. Therefore, the IR camera is not limited to the use of its spectral component, but also its 3D information derived from the structured light projection may be used. The 3D information may be less relevant for pulse and SpO2 measurement based on blood absorption variation (though still improve the face detection and tracking performance), but it can be advantageously used for respiration measurement based on the body surface movement.

Further, an adaptive control of the light emission may be provided, e.g. to obtain the desired narrow-band red sensing from the RGB camera for SpO2 measurement. Thus, instead of putting a narrow-band filter in front of the camera, the light source, e.g. the OLED display of a smartphone, may be used to create a narrow-band red illuminator that provides a strong red component for the RGB camera.

The main application of the present invention is in the field of contactless vital signs monitoring on a handheld device, including pulse-rate (variability), respiration rate, blood oxygen saturation, etc. The present invention improves the robustness of the vital signs extraction based on the property of the used reference, such as the time stability, spatial consensus, or the synchronization with other vital signs monitoring modalities. This is particularly beneficial for contactless monitoring, especially for customized applications on a handheld device like a vital signs camera APP. The contactless monitoring on a handheld device is highly relevant for home-based monitoring, daily-based vital signs tracking, wellness being and stress management. The present invention thus provides a full upgrade compared to known solutions used on conventional handheld devices and present significantly improved features, such as SpO2 measurement, monitoring in full-darkness and better robustness of vital signs extraction. Its application is expanded to include pulse-rate monitoring during fitness exercise (using NIR to increase motion robustness), driver monitoring in automotive (both day and night vision), baby motioning during sleep (full darkness).

In embodiments, the dual-cameras of a smartphone may be used to improve the robustness. 3D information of the NIR camera may be used to improve the face detection and tracking. 4-wavelength information (RGB+IR) may be used to improve the PPG extraction. Two cameras with slightly different view angle may be used to increase the measured skin area providing spatial redundancy.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for determining a vital sign of a subject, the device comprising:

   - a first camera (11) configured to acquire first images of the subject in a visible portion of the spectrum,
   - a second camera (12) configured to acquire second images of the subject in an infrared portion of the spectrum,
   - a visible light source (13) configured to emit visible light,
   - an infrared light source (14) configured to emit infrared light,
   - a processor (16) configured to determine a vital sign of the subject at least from the acquired first images,

   wherein the processor (16) is configured to determine SpO2 of the subject from the red portion of the spectrum of the first images and the infrared portion of the second images,
   wherein, in case SpO2 shall be determined as vital sign, the light emitted by the visible light source is controlled or filtered so that light in the red portion of the spectrum is emitted and/or the light sensed by the first camera is filtered so that light in the red portion of the spectrum is sensed and represented by the first images, and
   wherein the first camera, the second camera, the visible light source and the infrared light source are arranged on the same surface side of the device.

2. Device as claimed in claim 1,
   further comprising a controller (31) configured to control the visible light source to emit, in case SpO2 shall be determined as vital sign, light in the red portion of the spectrum.

3. Device as claimed in claim 1 or 2,
   further comprising a red filter (41) arranged in front of the first camera (11).

4. Device as claimed in claim 3,
   wherein the red filter (41) is configured to be switched on or off or to be arranged in front of or removed from the first camera.

5. Device as claimed in any one of the preceding claims,
   wherein the processor (16) is configured to extract the red portion of the spectrum from the first images.

6. Device as claimed in any one of the preceding claims,
further comprising a controller (31) configured to control the infrared light source (14).

7. Device as claimed in claim 6,
wherein the controller (31) is configured to control the infrared light source (14) to emit a grid pattern onto the subject, wherein the processor (16) is configured to derive 3D information from the second images and to use the derived 3D information for detection and/or tracking of a skin region of the subject and/or for detection of movement of a body portion of the subject's body.

8. Device as claimed in any one of claims 1 to 6,
wherein the processor (16) is configured to receive 3D information of the subject and to use the derived 3D information for detection and/or tracking of a skin region of the subject and/or for detection of movement of a body portion of the subject's body caused by respiration of the subject and/or ballistocardiographic motion.

9. Device as claimed in 7 or 8,
wherein the processor (16) is configured to register first and second images using the 3D information before using the first and/or second images to determine a vital sign.

10. Device as claimed in 9,
wherein the processor (16) is configured to use a global linear-model based pixel transformation or local non-linear block-to-block or pixel-to-pixel based pixel interpolation for registration of the first and second images.

11. Device as claimed in claim 2 or 6,
wherein the controller (31) is configured to adjust the spectrum, amplitude and/or modulation frequency of the light emitted by the visible light source and/or the infrared light source based on a quality metric of the derived vital sign.

12. Device as claimed in any one of the preceding claims,
wherein the visible light source is a display or touch screen.

13. Device as claimed in any one of the preceding claims,
wherein the device is a hand-held device, in particular a smartphone, pad, tablet or watch.

14. Method for determining a vital sign of a subject, the method comprising:

- controlling a first camera (11) to acquire first images of the subject in a visible portion of the spectrum,
- controlling a second camera (12) to acquire second images of the subject in an infrared portion of the spectrum,
- controlling a visible light source (13) to emit visible light,
- controlling an infrared light source (14), arranged on the same surface side of a device as the first camera, the second camera and the visible light source, to emit infrared light, and
- determining SpO2 of the subject from the red portion of the spectrum of the first images and the infrared portion of the second images, wherein the light emitted by the visible light source is controlled or filtered so that light in the red portion of the spectrum is emitted and/or the light sensed by the first camera is filtered so that light in the red portion of the spectrum is sensed and represented by the first images.

15. Computer program which comprises program code means for causing a computer to perform the steps of the method claimed in claim 14 when said computer program is carried out on a computer.

10

13

23

11

21

16

26

12

22

14

24

FIG.1

14  11  12  15

100

16

13

FIG.2

FIG.3

FIG.4

```
┌─────────────────────────────────────┐
│   stream first and second images     │ ~S10
└─────────────────────────────────────┘
                 │
                 ▼
┌─────────────────────────────────────┐
│   estimate stereo image / 3D         │ ~S12
│   information                        │
└─────────────────────────────────────┘
                 │
                 ▼
┌─────────────────────────────────────┐
│         detect / track ROI           │ ~S14
└─────────────────────────────────────┘
                 │
                 ▼
┌─────────────────────────────────────┐
│          register images             │ ~S16
└─────────────────────────────────────┘
                 │
                 ▼
┌─────────────────────────────────────┐
│       classify light condition       │ ~S18
└─────────────────────────────────────┘
                 │
                 ▼
┌─────────────────────────────────────┐
│        extract vital sign(s)         │ ~S20
└─────────────────────────────────────┘
                 │
                 ▼
┌─────────────────────────────────────┐
│       control light emission         │ ~S22
└─────────────────────────────────────┘
```

FIG.5

Europäisches Patentamt
European Patent Office
Office européen des brevets

# EUROPEAN SEARCH REPORT

Application Number

EP 19 21 6506

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/358332 A1 (WATANABE HISASHI [JP]) 8 December 2016 (2016-12-08) * paragraphs [0155] - [0162]; figures 7-10, 12 * * the whole document * | 1-15 | INV. A61B5/024 A61B5/11 A61B5/1455 A61B5/00 |
| X | WO 2019/157190 A1 (WALLACE ARTHUR [US]) 15 August 2019 (2019-08-15) * page 5, line 6 - page 8, line 22; figures 1-3 * * page 9, lines 13-14 * * the whole document * | 1-15 | |
| A | VAN GASTEL MARK ET AL: "Motion Robust Remote-PPG in Infrared", IEEE TRANSACTIONS ON BIOMEDICAL ENGINEERING, IEEE SERVICE CENTER, PISCATAWAY, NJ, USA, vol. 62, no. 5, 1 May 2015 (2015-05-01), pages 1425-1433, XP011578781, ISSN: 0018-9294, DOI: 10.1109/TBME.2015.2390261 [retrieved on 2015-04-17] * section II.D.1 on p. 1430; figure 8 * * the whole document * | 1-15 | |
| A | US 2018/333088 A1 (HOLZ CHRISTIAN [US] ET AL) 22 November 2018 (2018-11-22) * paragraphs [0014] - [0014]; figure 1 * | 12 | TECHNICAL FIELDS SEARCHED (IPC) A61B |

The present search report has been drawn up for all claims

| Place of search | Date of completion of the search | Examiner |
|---|---|---|
| The Hague | 23 June 2020 | Sarcia, Regis |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 19 21 6506

This annex lists the patent family members relating to the patent documents cited in the above-mentioned European search report.
The members are as contained in the European Patent Office EDP file on
The European Patent Office is in no way liable for these particulars which are merely given for the purpose of information.

23-06-2020

| Patent document cited in search report | Publication date | Patent family member(s) | | Publication date |
|---|---|---|---|---|
| US 2016358332 A1 | 08-12-2016 | CN | 106236060 A | 21-12-2016 |
| | | CN | 106236061 A | 21-12-2016 |
| | | JP | 2017000742 A | 05-01-2017 |
| | | JP | 2017000743 A | 05-01-2017 |
| | | US | 2016358011 A1 | 08-12-2016 |
| | | US | 2016358332 A1 | 08-12-2016 |
| WO 2019157190 A1 | 15-08-2019 | NONE | | |
| US 2018333088 A1 | 22-11-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015197383 A1

**Non-patent literature cited in the description**

- **W. WANG ; A. C. DEN BRINKER ; S. STUIJK ; G. DE HAAN.** Algorithmic principles of remote-PPG. *IEEE Transactions on Biomedical Engineering,* 2017, vol. 64 (7), 1479-1491

- **M. VAN GASTEL ; S. STUIJK ; G. DE HAAN.** New principle for measuring arterial blood oxygenation, enabling motion-robust remote monitoring. *Nature Scientific Reports,* November 2016